# EUROPÄISCHE PATENTSCHRIFT

(11) **EP 1 523 364 B1**
(45) Veröffentlichungstag und Bekanntmachung des Hinweises auf die Patenterteilung: **27.02.2008**
(21) Anmeldenummer: 03765038.9
(22) Anmeldetag: 18.07.2003
(51) Int. Cl.: A61M 13/00, A61M 1/16, G01F 1/32

(54) **VORRICHTUNG ZUR INSUFFLATION EINER KÖRPERHÖHLE MIT EINEM INSUFFLATIONSGAS**
DEVICE FOR INSUFFLATING AN INSUFFLATION GAS INTO A BODY CAVITY
DISPOSITIF D'INSUFFLATION D'UN GAZ D'INSUFFLATION DANS UNE CAVITE DE L'ORGANISME

(30) Priorität: 19.07.2002 DE 10233861
(43) Veröffentlichungstag der Anmeldung: 20.04.2005
(73) Patentinhaber: Storz Endoskop Produktions GmbH, 78532 Tuttlingen (DE)
(72) Erfinder: DIEMUNSCH, Pierre, F67000 Strasbourg (FR); NOVAK, Pavel, CH-8234 Stetten (CH)
(74) Vertreter: Weller, Wolfgang
(86) Internationale Anmeldenummer: PCT/EP2003/007809
(87) Internationale Veröffentlichungsnummer: WO 2004/009167

(56) Entgegenhaltungen:
- US-A- 5 098 375
- US-A- 5 328 458
- US-A- 5 810 759
- US-B1- 6 305 212

## Beschreibung

Die Erfindung betrifft eine Vorrichtung zur Insufflation einer Körperhöhle mit einem Insufflationsgas, mit einer Insufflationsvorrichtung mit einer Zuführleitung zur Zufuhr eines Insufflationsgases zu einer Körperhöhle.

Eine derartige Insufflationsvorrichtung ist beispielsweise aus dem Artikel "Die Laparoskopie in der Gynäkologie" von K. Semm in "Geburtshilfe und Frauenheilkunde", Heft 11, November 1967, bekannt. Mit Hilfe der Insufflationsvorrichtung, dem sogenannten Insufflator, wird ein Insufflationsgas, üblicherweise Kohlensäuregas (CO₂-Gas) in eine Körperhöhle eines Menschen oder Tieres, beispielsweise in die Bauchhöhle, eingebracht. Das Insufflationsgas bläht die Bauchhöhle auf, so daß dort der für eine Untersuchung, gegebenenfalls für eine Operation, mit Hilfe eines Endoskops erforderliche Betrachtungs- bzw. Operationsraum zwischen den inneren Organen und der Bauchdecke geschaffen wird.

Zusätzlich zu dem Insufflationsgas können sich in dem Abdominum weitere Gase ansammeln, beispielsweise Lachgas (N₂O) und/oder Methan (MH₄), wobei im Abdominum explosive Gasgemische entstehen können. Insbesondere im Zusammenhang mit elektrochirurgischen Maßnahmen kann es dabei zu Gasexplosionen kommen.

Lachgas wird zur Anästhesie verwendet. Aus der Blutbahn kann es in die intraabdominelle Kavität diffundieren. Beim Überschreiten einer Lachgaskonzentration von ca. 29% besteht eine erhöhte Explosionsgefahr des im abdominalen Bereich befindlichen Gasgemisches. Das Diffusionsverhalten von Lachgas im abdominalen Bereich von Schweinen ist aus dem Artikel "Nitrous Oxide Fraction in the Carbon Dioxide Pneumoperitoneum During Laparascopy Under General Inhalation Anesthesia in Pigs" von P. Diemunsch, Klaus D. Torp, T. Van Dorsselaer, D. Mutter, A. M. Diemunsch, R. Schaeffer, G. Teller und A. Van Dorselaer bekannt. In dem Artikel ist beschrieben, wie mit Hilfe eines Katheters sowie einer gasdichten Spritze im Abständen von je zehn Minuten Proben aus abdominalen Räumen von Schweinen entnommen worden sind. Zur Anästhesie wurde Lachgas verwendet. Die abdominalen Räume wurden mit Kohlensäuregas aufgebläht. Dabei wurde über einen besonders langen Beobachtungszeitraum das Verhalten der Lachgaskonzentration im Abdominum beobachtet. Nach etwa zwei Stunden ist demnach die kritische Grenze von 29% Lachgaskonzentration erreicht. Nach ca. neun Stunden steigt die Lachgaskonzentration nur noch unwesentlich über einen Wert von ca. 66% an.

Für eine wissenschaftliche Untersuchung mag das in dem Artikel beschriebene Meßverfahren mit einem Katheder und einer gasdichten Spritze brauchbar sein. Für eine typische endoskopische Maßnahme, bei der der Patient möglichst schonend behandelt werden soll und bei der die Behandlungsperson ihre volle Konzentration dem Patienten zuwenden sollte, ist eine derartige Vorgehensweise jedoch ungeeignet.

Ein weiteres Problem bei intraabdominellen Eingriffen ist, daß der Darm des Patienten versehentlich verletzt oder perforiert werden kann. Vor allem bei Verwendung von Elektrochirurgie-Instrumenten ist diese Gefahr sehr groß. Die Verletzung des Darmes führt zum Entweichen von Darmgasen, die Methan enthalten. Das Methan kann zu Explosionen führen. Ein weitaus größeres Problem ist allerdings, daß die Verletzung des Darmes während des Eingriffes nicht erkannt wird. Postoperativ kann dies zu ernsthaften Komplikationen beim Patienten, sogar bis zu dessen Tode, führen. Eine Analyse der Gase im abdominalen Bereich in der aus dem oben genannten Artikel bekannten Art und Weise kommt jedoch auch in Bezug auf Methan aufgrund der komplizierten Probenentnahme kaum in Frage.

Die US 5,328,458 beschreibt eine Vorrichtung zum Insufflieren einer Körperhöhle mit einem Insufflationsgas. Die in diesem Dokument beschriebene Vorrichtung weist Vorratsbehälter für ein Insufflationsgas auf, die über verschiedene Ventile mit einer Kanüle zum Einleiten des Insufflationsgases in eine Körperhöhle verbunden sind. Die Vorrichtung weist außerdem zumindest einen Drucksensor auf, der den in der Körperhöhle vorhanden Druck des Insufflationsgases misst. Weiterhin weist die Vorrichtung eine arithmetische Einheit und eine Regeleinheit auf. Mit Hilfe der arithmetischen Einheit und der Regeleinheit wird der Druck in der Körperhöhle auf einem vorgegebenen Wert gehalten.

Die US 6,305,212 B1 beschreibt eine Vorrichtung zur Echtzeitgasanalyse. In dieser Vorrichtung werden die Konzentrationen von bekannten Bestandteilen einer Mischung von Gasen durch Messen der Eigenschaften der Mischung und Lösen eines Gleichungssatzes bestimmt. Bei den gemessenen Werten handelt es sich um Temperatur, Absolutdruck, Durchflussrate, Druckverlust durch eine Kapillare und Druckverlust durch eine Öffnung.

Die US 5,801,759 beschreibt ein System zur Regelung des Gasaustausches in einem extrakorporalen Blutkreislauf. Ein solcher extrakorporaler Blutkreislauf wird z.B. in einer Herz-Lungen-Maschine eingesetzt. Hierbei wird der Gehalt an Kohlendioxid und Sauerstoff im Blut gemessen, so dass den Patienten stets eine ausreichende Menge Sauerstoff zugeführt wird, während die Kohlendioxid-Konzentration nicht über einen gewissen Wert hinaus steigt. Das Blut im extrakorporalen Blutkreislauf wird hierbei laufend untersucht und z.B. die Zugabe von Sauerstoff automatisch geregelt.

Die US 5,098,375 beschreibt eine Vorrichtung zur Insufflation einer Körperhöhle mit Rückführung des Insufflationsgases. Die Vorrichtung weist hierzu eine Kanüle auf, mit der ein Insufflationsgas in eine Körperhöhle eingeleitet werden kann. Die Vorrichtung weist weiterhin eine Einheit auf, die das Insufflationsgas aus einer Körperhöhle abführt und nach Reinigung in einem Sterilfilter wieder in die Körperhöhle einleitet. Die Vorrichtung weist weiter einen Drucksensor auf, der das Einleiten bzw. Abführen des Insufflationsgases regelt.

Es ist daher Aufgabe der Erfindung, etwaige Komplikationen frühzeitig und zuverlässig zu ermitteln, die bei einem Eingriff auftreten können, bei dem ein Insufflationsgas in eine Körperhöhle, insbesondere in den abdominalen Raum, eines Menschen oder Tieres insuffliert wird.

Erfindungsgemäß wird die Aufgabe dadurch gelöst, daß bei der eingangs genannten Vorrichtung die Insufflationsvorrichtung eine Abführleitung zum Abführen eines Meßgases aus der Körperhöhle aufweist, und daß sie eine Meßvorrichtung zur Messung eines zusätzlich zu dem Insufflationsgas in dem Meßgas enthaltenen Zusatzstoffes und zur Ausgabe eines Meßsignals in Abhängigkeit von dem Zusatzstoff aufweist, wobei die Meßvorrichtung als Gassensor ausgebildet ist.

Ein Grundgedanke der Erfindung ist, die Insufflation der Körperhöhle mit einem Insufflationsgas und die Analyse des dort befindlichen Gasgemisches kombiniert durchzuführen. Die Insufflationsvorrichtung und die Meßvorrichtung kommen gemeinsam zum Einsatz. Die den Eingriff durchführende Person ist während des Eingriffes in der Körperhöhle, insbesondere in einem abdominalen Raum, stets über die dort vorhandenen Gas-Zustände im Bilde, wodurch kritische Situationen während und nach dem Eingriff vermieden werden können.

Beispielsweise kann ermittelt werden, ob sich möglicherweise im Abdominum ein zündfähiges Gasgemisch angesammelt hat. Dementsprechend kann beispielsweise die Zufuhr des Insufflationsgases erhöht und zugleich die Körperhöhle entlüftet werden, wodurch die Konzentration brennbarer Gase im Abdominum absinkt. Eine mögliche Explosion läßt sich auf diese Weise zuverlässig verhindern.

Eine etwaige Verletzung des Darmes des Patienten kann noch während des Eingriffes erkannt werden. Man kann sofort Gegenmaßnahmen einleiten, wodurch postoperative Komplikationen, möglicherweise sogar der Tod des Patienten, vermieden werden können.

Ein weiterer wichtiger Aspekt der Erfindung ist eine Entlüftung der Körperhöhle durch die Abfuhr des Meßgases. Dadurch wird der Bildung zündfähiger Gasgemische in der Körperhöhle vorgebeugt.

In einer Ausgestaltung der Erfindung weist die Insufflationsvorrichtung eine Insufflationskanüle auf, in der eine Zuführ-Leitung zum Zuführen des Insufflationsgases zu der Körperhöhle und eine Abführ-Leitung zum Abführen des Meßgases aus der Körperhöhle vorhanden ist.

Diese Maßnahme hat den handhabungsfreundlichen Vorteil, daß lediglich eine einzige Insufflationskanüle in die Körperhöhle einzubringen ist. Der Patient wird nur an einer einzigen Stelle durch das Einbringen der Insufflationskanüle tangiert. Prinzipiell wäre es aber auch möglich, daß zum Zuführen des Insufflationsgases zu der Körperhöhle und zum Abführen des Meßgases aus der Körperhöhle jeweils separate Kanülen verwendet werden.

In einer weiteren Ausgestaltung der Erfindung weist die Insufflationsvorrichtung einen Schlauch auf, in dem die Zuführ-Leitung zum Zuführen des Insufflationsgases zu der Körperhöhle und die Abführ-Leitung zum Abführen des Meßgases aus der Körperhöhle vorhanden sind.

Auch diese Maßnahme ist in Bezug auf die Handhabung vorteilhaft. An den Schlauch, bei dem es sich beispielsweise um einen mehrlumigen Schlauch handelt, können mehrere Kanülen angeschlossen werden, beispielsweise eine Zuführ-Kanüle zum Zuführen des Insufflationsgases zu der Körperhöhle und eine AbführKanüle zum Abführen des Meßgases aus der Körperhöhle. Besonders vorteilhaft jedoch ist die Verwendung einer "kombinierten" Insufflationskanüle gemäß der vorgenannten Ausgestaltung der Erfindung, bei der eine Zuführ-Leitung und eine Abführ-Leitung vorgesehen sind.

In einer weiteren Ausgestaltung der Erfindung weist die Vorrichtung eine Warnvorrichtung zur Ausgabe einer Warnmeldung beim Überschreiten eines vorbestimmten Grenzwertes des von der Meßvorrichtung erfaßten Zusatzstoffes auf.

Durch diese Maßnahme erhält die Behandlungsperson unmittelbar eine Warnmeldung, wenn in der behandelten Körperhöhle eine kritische Situation auftritt, beispielsweise wenn sich ein explosives Gasgemisch gebildet hat oder der Darm des Patienten verletzt worden ist.

Die folgenden Ausgestaltungen der Erfindung betreffen vorteilhafte Ausführungsformen der Meßvorrichtung.

In einer weiteren Ausgestaltung der Erfindung ist der Gassensor als Gas-Chromatograph ausgebildet. Bei dem Gas-Chromatographen handelt es sich beispielsweise um einen Kapillar-Gas-Chromatographen. Ein Gas-Chromatograph ermöglicht eine besonders genaue Analyse des Meßgases.

In einer weiteren Ausgestaltung der Erfindung ist der Gassensor als Massenspektrometer ausgebildet.

Auch diese Ausgestaltung der Erfindung ermöglicht eine exakte Analyse des Meßgases.

In einer weiteren Ausgestaltung der Erfindung ist der Gassensor zu einer Erfassung, ob das Meßgas brennbar ist, ausgestaltet.

Mit dieser Maßnahme lassen sich wirkungsvoll explosive Gasgemische in der behandelten Körperhöhle vermeiden. Dabei ist es möglich, daß die Meßvorrichtung nicht Art und Konzentration brennbarer Gase in dem Meßgas im Detail ermittelt, sondern lediglich, ob das Meßgas insgesamt brennbar ist.

In einer weiteren Ausgestaltung der Erfindung ist der Gassensor zur Erfassung von Methan (CH₄) und/oder Lachgas (N₂O) ausgestaltet.

Wie bereits erläutert, ermöglicht es die Analyse von Methan in dem Meßgas, mögliche Darmverletzungen frühzeitig zu erkennen. Methan ist in erster Linie dafür verantwortlich, daß es zu Verbrennungen oder gar Explosionen in der Körperhöhle kommen kann.

Es versteht sich, daß die Meßvorrichtung auch zur Erfassung weiterer Zusatzstoffe ausgestaltet sein kann, beispielsweise verschiedenartiger Darmgase, Sauerstoff (O₂), dem Insufflationsgas, beispielsweise Kohlensäuregas (CO₂), Wasserstoff oder dergleichen. Auch eine Erfassung von in dem Meßgas enthaltenen Arzneimitteln, die dem Patienten verabreicht worden sind, ist möglich.

In einer weiteren Ausgestaltung der Erfindung weist die erfindungsgemäße Vorrichtung eine Druck-Meßvorrichtung zur Messung des Druckes des der Insufflationskanüle zugeführten Insufflationsgases und/oder des Meßgases auf.

Die Druck-Meßvorrichtung kann beispielsweise in der Insufflationsvorrichtung enthalten sein. Vorzugsweise jedoch bildet sie einen Bestandteil der Meßvorrichtung. Prinzipiell wäre es aber auch möglich, sowohl in der Insufflationsvorrichtung als auch in der Meßvorrichtung jeweils eine Druck-Meßvorrichtung vorzusehen. Eine Druck-Meßvorrichtung ist insbesondere in Bezug auf die Insufflationsvorrichtung vorteilhaft. Anhand der von der Druck-Meßvorrichtung ermittelten Meßergebnisse kann die Insufflationsvorrichtung die Druckverhältnisse in der Körperhöhle überwachen und gegebenenfalls mehr oder weniger Insufflationsgas der Körperhöhle zuführen.

In einer weiteren Ausgestaltung der Erfindung weist die erfindungsgemäße Vorrichtung eine Mengen-Meßvorrichtung zur Messung der Menge des Meßgases auf.

Eine Mengen-Meßvorrichtung kann bei der Insufflationsvorrichtung und/oder bei der Meßvorrichtung vorgesehen sein. Genauso wie eine Druck-Meßvorrichtung gemäß der vorgenannten Ausgestaltung der Erfindung eignet sich eine Mengen-Meßvorrichtung zur Steuerung und Überwachung der Druck- und Mengenverhältnisse des Insufflationsgases in der Körperhöhle.

In einer weiteren Ausgestaltung der Erfindung ist die Insufflationsvorrichtung zur Steuerung der Menge und/oder des Drucks des der Körperhöhle zuzuführenden Insufflationsgases ausgestaltet.

Diese Maßnahme ist insbesondere im Zusammenhang mit den beiden vorgenannten Maßnahmen vorteilhaft. Die Insufflationsvorrichtung wertet dabei die Meßergebnisse der Mengen-Meßvorrichtung und/oder der Druck-Meßvorrichtung aus und steuert dementsprechend die Zufuhr des Insufflationsgases in Richtung der Körperhöhle.

In einer weiteren Ausgestaltung der Erfindung ist die Insufflationsvorrichtung zur Steuerung der Zufuhr des Insufflationsgases zu der Körperhöhle in Abhängigkeit von dem durch die Meßvorrichtung erzeugten, den Zusatzstoff betreffenden Meßsignal ausgestaltet.

Somit kann in vorteilhafter Weise beispielsweise die Zufuhr des Insufflationsgases erhöht werden, um den Anteil brennbarer Gase in der Körperhöhle im Verhältnis zu dem Insufflationsgas zu senken.

In diesem Zusammenhang soll auch erwähnt sein, daß die Insufflationsvorrichtung prinzipiell auch zur Steuerung der Abfuhr des Insufflationsgases aus der Körperhöhle in Abhängigkeit des von der Meßvorrichtung erzeugten Meßsignals ausgestaltet sein kann. Auch diese Maßnahme ist geeignet, um eine möglicherweise unerwünschte Gasgemisch-Situation in der Körperhöhle zu verbessern.

Die im folgenden erläuterten, besonders bevorzugten Ausführungsformen der Erfindung betreffen die Zufuhr von Insufflationsgas zur Körperhöhle bzw. die Abfuhr von Meßgas aus der Körperhöhle.

In einer weiteren Ausgestaltung der Erfindung ist die Insufflationsvorrichtung zum Zuführen eines im wesentlichen kontinuierlichen Insufflationsgasstromes zu der Körperhöhle ausgestaltet.

Prinzipiell wäre es zwar möglich, das Insufflationsgas auch diskontinuierlich der Körperhöhle zuzuführen, beispielsweise in Form von jeweils einem oder mehreren Pumpstößen. Derartige Pumpstöße sind jedoch häufig mit Geräusch verbunden, was die Behandlungsperson bei dem endoskopischen Eingriff stört. Demgegenüber ist ein kontinuierliches Zuführen des Insufflationsgasstromes zu der Körperhöhle geräuscharm, wenn nicht gar geräuschlos. Zudem werden Vibrationen, die möglicherweise auch den Patienten belasten, verringert oder gar vermieden.

In einer weiteren Ausgestaltung der Erfindung ist die Insufflationsvorrichtung zum Abführen eines im wesentlichen kontinuierlichen Meßgasstromes ausgestaltet.

Hier gilt im Grunde genommen das selbe wie bei der vorgenannten Maßnahme, nämlich daß ein kontinuierlicher Gasstrom vergleichsweise geräuscharm ist und eventuelle mechanische Belastungen von Patient und Insufflationsvorrichtung vermindert.

"Kontinuierlich" im Sinne der beiden vorgenannten Maßnahmen heißt, daß der jeweilige Gasstrom nicht oder nur selten unterbrochen wird. Allerdings kann das geförderte Gasvolumen, also der Gasstrom, dabei durchaus vergrößert oder verkleinert werden.

In einer weiteren Ausgestaltung der Erfindung ist der Meßgasstrom zur Entlüftung der Körperhöhle geeignet.

Es ist also eine gezielte Gasleckage vorgesehen, um die Körperhöhle zu entlüften. Durch die Entlüftung wird einer Bildung eines eventuell explosiven Gasgemisches in der Körperhöhle vorgebeugt. Es versteht sich, daß die gezielte Entlüftung der Körperhöhle vorteilhaft mit einer entsprechend hohen Zufuhr von Insufflationsgas zur Körperhöhle zu kombinieren ist. In der Körperhöhle wird dabei das abgeführte Meßgas durch ein frisch zugeführtes Insufflationsgas ersetzt. Es hat sich gezeigt, daß das Abführen von etwa einem halben Liter bis einem Liter Meßgas pro Minute zweckmäßig ist. In diesem Zusammenhang soll auch erwähnt werden, daß es sich bei dem Meßgas durchaus um reines Insufflationsgas handeln kann, nämlich dann, wenn beispielsweise kein Lachgas, Darmgas oder sonstiger Stoff in das in die Körperhöhle insufflierte Insufflationsgas diffundiert.

Ein Mindestgasfluß zur Entlüftung der Körperhöhle, insbesondere ein kontinuierlicher Meßgasstrom, gemäß der vorteilhaften Ausgestaltung der Erfindung erlaubt es, der Körperhöhle einen vergleichsweise hohen Insufflationsgasstrom zuzuführen. Das Insufflationsgas kann dabei unter einem verhältnismäßig hohen Druck stehen. Man kann deshalb dünnere Insufflationsschläuche und dünnere Insufflationskanülen verwenden, die leichter handhabbar sind und den Patienten weniger beanspruchen. Eine erfindungsgemäße Insufflationskanüle bzw. ein erfindungsgemäßer Insufflationsschlauch, bei denen jeweils sowohl eine Zuführ-Leitung für das Insufflationsgas sowie eine Abführ-Leitung für das Meßgas vorgesehen sind, sind daher - obwohl zwei oder mehr Leitungen vorgesehen sind - vergleichsweise dünn. Eine derartige mehrkanalige Insufflationskanüle bzw. ein derartiger mehrkanaliger Insufflationsschlauch sind nicht oder allenfalls unwesentlich dicker als eine konventionelle Insufflationskanüle bzw. ein konventioneller Insufflationsschlauch, bei denen nur jeweils eine Leitung vorgesehen ist. Ein Insufflator mit einer mehrkanaligen Insufflationskanüle und/oder einem mehrkanaligen Insufflationsschlauch, die zur Zufuhr eines Insufflationsgases zu einer Körperhöhle und zugleich zur Abfuhr eines Entlüftungsgases aus der Körperhöhle ausgestaltet sind, stellen im Grunde genommen bereits eine eigene Erfindung dar.

In einer weiteren Ausgestaltung der Erfindung weist die erfindungsgemäße Vorrichtung eine Abführpumpe zum Abführen des Meßgases auf.

Eine derartige Abführpumpe kann sowohl bei der Insufflationsvorrichtung als auch bei der Meßvorrichtung vorgesehen sein. Die Abführpumpe, bei der es sich beispielsweise um eine kleine Saugpumpe handeln kann, sorgt für einen Mindestabfluß von Meßgas aus der Körperhöhle. Das Fördervolumen der Pumpe kann einstellbar sein.

Es versteht sich, daß die vorstehend genannten und die nachstehend noch zu erläuternden Merkmale nicht nur in den angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand von ausgewählten Ausführungsbeispielen im Zusammenhang mit den beiliegenden Zeichnungen näher beschrieben und erläutert. Es zeigen:
- Fig. 1: teilweise stark schematisiert eine perspektivische Ansicht eines ersten Ausführungsbeispiels einer erfindungsgemäßen Vorrichtung zur Insufflation einer Körperhöhle mit einem Insufflationsgas,
- Fig. 2: einen Schnitt längs einer Linie II-II eines Insufflationsschlauches der Vorrichtung gemäß Fig. 1,
- Fig. 3: einen Schnitt längs einer Linie III-III einer Insufflationskanüle der Vorrichtung gemäß Fig. 1, und
- Fig. 4: teilweise stark schematisiert eine perspektivische Ansicht eines zweiten Ausführungsbeispiels einer erfindungsgemäßen Vorrichtung zur Insufflation einer Körperhöhle mit einem Insufflationsgas.

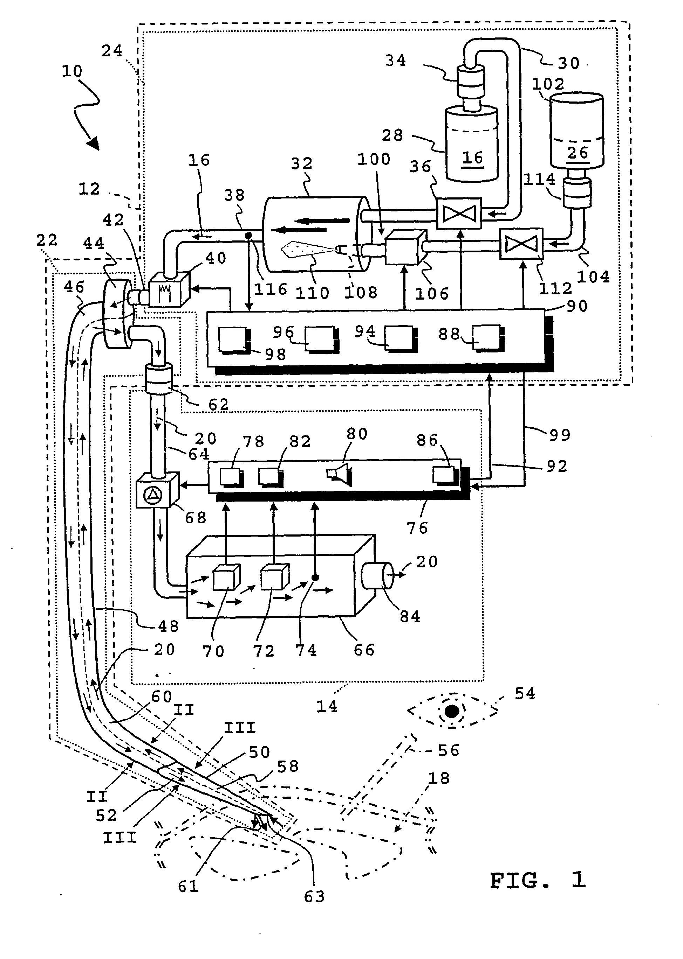

In den Figuren 1 bis 3 ist eine Vorrichtung zur Insufflation einer Körperhöhle mit einem Insufflationsgas in ihrer Gesamtheit mit der Bezugsziffer 10 versehen.

Die Vorrichtung 10 folgt einem modularen Konzept, bei dem eine erfindungsgemäße Meßvorrichtung 14 an eine erfindungsgemäße Insufflationsvorrichtung 12 anschließbar ist. In der Zeichnung ist die Meßvorrichtung 14 im an die Insufflationsvorrichtung 12 angeschlossenen Zustand gezeigt.

Die Insufflationsvorrichtung 12 dient zur Zufuhr eines Insufflationsgases 16 zu einer Körperhöhle 18, beispielsweise dem abdominalen Bereich, eines Menschen oder Tieres. Zudem führt die Insufflationsvorrichtung 12 ein Meßgas 20 aus der Körperhöhle 18 ab und führt dieses Meßgas 20 der Meßvorrichtung 14 zu. Zum Führen beider Gase 16, 20 ist bei der Insufflationsvorrichtung 12 eine kombinierte Zu- und Abführvorrichtung 22 vorgesehen, die im folgenden zur Vereinfachung als "Zuführvorrichtung 22" bezeichnet wird. Die Zuführvorrichtung 22 ist an eine Aufbereitungsvorrichtung 24 angeschlossen, mit der sie lösbar verbindbar ist. Die Aufbereitungsvorrichtung 24 dient zur Zufuhr des Insufflationsgases 16 zu der Zuführvorrichtung 22. Mit der Aufbereitungsvorrichtung 24 sind Menge und Druck des der Zuführvorrichtung 22 zugeführten Insufflationsgases 16 steuerbar. Zudem bereitet die Aufbereitungsvorrichtung 24 das Insufflationsgas 14 auf. Beim Ausführungsbeispiel belädt die Aufbereitungsvorrichtung 24 das Insufflationsgas 16 mit einer Befeuchtungsflüssigkeit 26.

Die Meßvorrichtung 14 dient zur Analyse des Meßgases 20. Die Meßvorrichtung 14 analysiert das Meßgas 20 und ermittelt einen oder mehrere, in dem Meßgas 20 enthaltene Zusatzstoffe, beispielsweise Lachgas, Methan oder dergleichen.

Im folgenden wird die Funktionsweise der Vorrichtung 10 näher erläutert:

Aus einem vorliegend kartuschenartigen Vorratsbehälter 28 strömt das Insufflationsgas 16, bei dem es sich beispielsweise um Kohlensäuregas (CO₂) handelt, über eine Leitung 30 zu einer Aufbereitungskammer 32 der Insufflationsvorrichtung 12. Der Vorratsbehälter 28 ist über eine lösbare Verbindung, beispielsweise eine Schraub- oder Bajonett-Verbindung 34 mit der Leitung 30 verbunden. An der Leitung 30 ist eine Ventilanordnung 36 angeordnet, mit der der in die Aufbereitungskammer 32 einströmende Insufflationsgasstrom einstellbar ist.

Aus der Aufbereitungskammer 32 strömt das Insufflationsgas 16 über eine Leitung 38 zu einer Heizung 40. Die Heizung 40 dient zur Erwärmung des Insufflationsgases 16, so daß dieses optimal an die mikroklimatischen Verhältnisse in der Körperhöhle 18 angepaßt ist. Die Heizung 40 ist mittels eines Anschlußstücks 42 mit einer Anschlußvorrichtung 44 der Zuführvorrichtung 22 lösbar verbunden. Das Anschlußstück 42 sowie die Anschlußvorrichtung 44 bilden beispielsweise eine Schraub- und/oder Steckverbindung.

Von der Anschlußvorrichtung 44 strömt das Insufflationsgas 16 über eine Zuführ-Leitung 46 eines Schlauches 48 zu einer Insufflationskanüle 50. Die Insufflationskanüle 50 durchströmt das Insufflationsgas 16 durch eine Zuführ-Leitung 52 und tritt aus der Insufflationskanüle 50 an deren vorderem, dem Schlauch 48 abgewandten Ende in die Körperhöhle 18 aus. Dort bildet das Insufflationsgas 16 einen aufgeblähten Raum, so daß eine Behandlungsperson 54 mit Hilfe eines Endoskops 56 Untersuchungen, Operationen oder dergleichen durchführen kann.

Bei einem derartigen Eingriff wird beispielsweise Lachgas (N₂O) zur Anästhesie des Patienten verwendet. Ferner ist es denkbar, daß der Darm des Patienten bei der Behandlung verletzt wird, wodurch Darmgase, insbesondere Methan, in die Körperhöhle 18 austreten. Diese in der Figur nicht näher bezeichneten Zusatzstoffe können sich mit dem insufflierten Insufflationsgas 16 vermischen. Ferner kann das in der Körperhöhle 18 befindliche Insufflationsgas 16 mit partikelartigen und/oder flüssigen Zusatzstoffen beladen sein, beispielsweise mit sich in der Körperhöhle 18 befindlicher Feuchtigkeit. Teilweise wird das sich in der Körperhöhle 18 befindliche Gas mit Hilfe der Zuführvorrichtung 22, bei der es sich insoweit um eine Abführvorrichtung handelt bzw. die eine Abführvorrichtung enthält, aus der Körperhöhle 18 zur Meßvorrichtung 14 abgeführt. Das abgeführte Gas bildet das Meßgas 20.

Das Meßgas 20 strömt über eine Abführ-Leitung 58 der Insufflationskanüle 50 zu einer Abführ-Leitung 60 des Schlauches 48, bei dem es sich beispielsweise um einen mehrlumigen Schlauch handelt. Die Abführ-Leitung 60 mündet in der Anschlußvorrichtung 44, mit der die Meßvorrichtung 14 über ein Anschlußstück 62 der Meßvorrichtung 14 verbunden ist. Das Anschlußstück 62 und die Anschlußvorrichtung 44 sind beispielsweise miteinander verschraubt und/oder ineinander gesteckt.

An der Anschlußvorrichtung 44 ist ein Auslaß 61 angeordnet, der mit einem Einlaß 63 des Anschlußstückes 62 in Verbindung steht. Das Meßgas 20 durchströmt den Auslaß 61 sowie den Einlaß 63. Das Meßgas 20 strömt von der Anschlußvorrichtung 44 über das Anschlußstück 62 sowie über eine Leitung 64 zu einer Meßkammer 66 der Meßvorrichtung 14. An der Leitung 64 ist eine Abführpumpe 68 angeordnet, die das Meßgas 20 ansaugt und in die Meßkammer 66 pumpt. Die Abführpumpe 68 fördert einen kontinuierlichen Meßgasstrom aus der Körperhöhle 18 zur Meßkammer 66.

Es versteht sich, daß die Abführpumpe 68 prinzipiell auch in einem diskontinuierlichen Betriebsmodus, z.B. in einem Pulsbetrieb oder dergleichen, betrieben werden kann.

Die Meßvorrichtung 14 weist eine Additiv-Meßvorrichtung 70, eine Mengen-Meßvorrichtung 72 sowie eine Druck-Meßvorrichtung 74 auf. Die Vorrichtungen 70, 72, 74 sind an der Meßkammer 66 angeordnet, wobei es im wesentlichen darauf ankommt, daß sie dort mit dem Meßgas 20 in Berührung kommen.

Die Additiv-Meßvorrichtung 70 enthält beispielsweise einen Gassensor, einen Gas-Chromatographen und/oder ein Massenspektrometer. Es versteht sich, daß auch nur eines der vorgenannten Instrumente bei der Additiv-Meßvorrichtung 70 vorhanden sein kann. In einer einfachen Ausführungsform ist beispielsweise ein Gassensor vorhanden, der ermittelt, ob das Meßgas 20 brennbar ist und/oder eine explosive Konsistenz aufweist. In dem Meßgas 20 können zusätzlich zu dem Insufflationsgas 16 Zusatzstoffe enthalten sein, beispielsweise Lachgas, Methan oder dergleichen, die von der Additiv-Meßvorrichtung 70 ermittelbar sind.

Die Mengen-Meßvorrichtung 72 sowie die Druck-Meßvorrichtung 74 messen die Menge bzw. den Druck des Meßgases 20. Anhand der so ermittelten Meßwerte sind die Mengen- bzw. Druck-Verhältnisse in der Körperhöhle 18 ermittelbar. Die ermittelten Meßwerte können aber auch dazu dienen, die Zufuhr des Insufflationsgases 16 zur Körperhöhle 18 zu steuern und zu überwachen. Eine Auswertevorrichtung 76 der Meßvorrichtung 14 dient zur Auswertung der von den Meßvorrichtungen 70, 72, 74 ermittelten Meßwerte.

Die Auswertevorrichtung 76 weist beispielsweise als Ausgabemittel ein Display 78 sowie einen Lautsprecher 80 auf, mit denen die von den Vorrichtungen 70, 72, 74 ermittelten Werte ausgebbar sind. Der Lautsprecher 80 dient beispielsweise zur Ausgabe eines Warntones oder einer sonstigen Warnmeldung, wenn ein von der Additiv-Meßvorrichtung 70 erfaßter Meßwert einen vorbestimmten Grenzwert überschreitet, beispielsweise wenn das Meßgas 20 explosiv ist. Zur Eingabe derartiger Grenzwerte oder für sonstige Bedienhandlungen dienen Eingabemittel 82 der Auswertevorrichtung 76, die beispielsweise Bedientasten oder dergleichen enthalten. Aus der Meßkammer 66 kann das Meßgas 20 durch einen Auslaß 84 ausströmen.

Am Auslaß 84 kann eine nicht dargestellte Ventilanordnung zum Verschließen des Auslasses 84 angeordnet sein. Jedenfalls ist die Körperhöhle 18 mit Hilfe der Zu- bzw. Abführvorrichtung 22 sowie der Meßvorrichtung 14 entlüftbar.

Die Auswertevorrichtung 76 weist eine Sendevorrichtung 86 auf, mit der sie Meldungen an eine Empfangsvorrichtung 88 einer Steuervorrichtung 90 der Insufflationsvorrichtung 12 übermitteln kann. Die Vorrichtungen 86, 88 sind beispielsweise über eine oder mehrere elektrische Leitungen sowie eine nicht dargestellte lösbare Steckverbindung miteinander verbunden. Bei den Signalen kann es sich prinzipiell um jedes der von Meßvorrichtungen 70, 72, 74 erzeugten Meßsignale handeln. Vorliegend wird z.B. ein von der Additiv-Meßvorrichtung 70 erzeugtes Meßsignal 92 an die Steuervorrichtung 90 übertragen. Das Meßsignal 92 ist von einem oder mehreren Zusatzstoffen abhängig, die in dem Meßgas 20 zusätzlich zu dem Insufflationsgas 16 enthalten sind, z.B. von einem Lachgasanteil in dem Meßgas 20.

Die Steuervorrichtung 86 dient zur Steuerung und Überwachung der Insufflationsvorrichtung 12. Sie weist Eingabemittel 94, beispielsweise Bedientasten, sowie Ausgabemittel 96, beispielsweise ein Display, Leuchtdioden oder dergleichen auf. Im vorliegenden Fall handelt es sich bei der Steuervorrichtung 90, wie im übrigen auch bei der Auswertevorrichtung 76, um eine Mikroprozessor-Steuerung. Lediglich bei der Steuervorrichtung 90 ist ein Mikroprozessor 98 schematisch dargestellt.

Die Steuervorrichtung 90 steuert die Ventilanordnung 36 zur Beeinflussung des der Körperhöhle 18 zuzuführenden Insufflationsgases 16 an.

Zudem steuert und überwacht die Steuervorrichtung 90 eine Befeuchtungsvorrichtung 100 zum Befeuchten des Insufflationsgases 16. Neben der Aufbereitungskammer 32 gehört ein kartuschenartiger Vorratsbehälter 102, aus dem die Befeuchtungsflüssigkeit 26 über eine Leitung 104 zu einer Einspritzvorrichtung 106 gelangt zu der Befeuchtungsvorrichtung 100. Die Einspritzvorrichtung 106 weist einen in die Aufbereitungskammer 32 mündenden Einspritzauslaß 108 auf. Die Einspritzvorrichtung 106 sprüht über den Einspritzauslaß 106 einen Befeuchtungsnebel 110 ein, der von dem Insufflationsgas 16 mitgenommen und zur Körperhöhle 18 geführt wird. Dort sorgt die Befeuchtungsflüssigkeit 16 für vorteilhafte mikroklimatische Verhältnisse, die zu einer Verringerung postoperativer Komplikationen führen. An der Leitung 104 ist eine Ventilanordnung 112 angeordnet, mit der der Fluß der Befeuchtungsflüssigkeit 16 in der Leitung 104 einstellbar ist. Der Vorratsbehälter 102 ist über eine lösbare Verbindung 116, beispielsweise eine Schraub- und/oder Steckverbindung, mit der Leitung 104 verbunden.

An der Leitung 38 ist ein Sensor 116 angeordnet, der die Menge sowie den Druck des von der Insufflationsvorrichtung 12 zur Körperhöhle 18 geförderten Insufflationsgases 16 erfaßt. Anhand der von dem Sensor 116 an die Steuervorrichtung 90 übermittelten Meßwerte kann diese die Menge des zur Körperhöhle 18 geförderten Insufflationsgases 16 steuern. Zusätzlich wertet die Steuervorrichtung 90 hierbei das von der Meßvorrichtung 14 gemeldete Meßsignal 92 aus. Wenn sich beispielsweise die Konzentration von einem oder mehreren in dem Meßgas 20 enthaltenen Zusatzstoffen in unerwünschter Weise erhöht, kann die Steuervorrichtung 90 den Strom des Insufflationsgases 16 zur Körperhöhle 18 erhöhen.

Dabei ist es prinzipiell möglich, daß die Steuervorrichtung 90 die Meßvorrichtung 14 in vorteilhafter Weise ansteuert: Beispielsweise kann die Steuervorrichtung 90 die Abführpumpe 68 in Abhängigkeit vom jeweils zur Körperhöhle 18 geförderten Insufflationsgasstroms ansteuern, wobei sie über eine Steuerleitung 99 entsprechende Steuersignale sendet. Wird mehr Insufflationsgas 16 zur Körperhöhle 18 gepumpt, erhöht die Abführpumpe 68 entsprechend ihre Fördermenge und umgekehrt.

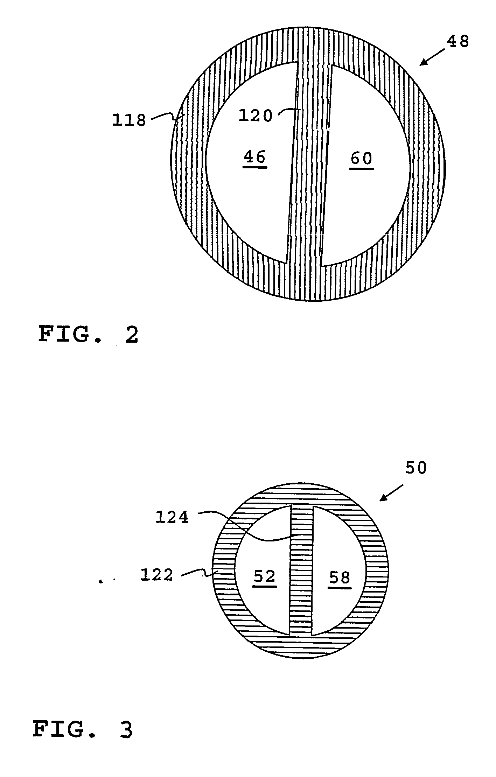

Aus der Schnittdarstellung von Fig. 2 ist erkennbar, daß der Schlauch 48 die beiden voneinander getrennten Leitungen 46 und 60 aufweist. Der Schlauch 60 besteht aus flexiblem Material, beispielsweise Gummi oder Kunststoff. Die Leitungen 46, 60 sind innerhalb eines Außenmantels 118 ausgebildet und durch eine Trennwand 120 voneinander getrennt. Der Schlauch 48 weist vorliegend einen im wesentlichen kreisrunden Querschnitt auf. Die Leitungen 46 und 60 haben jeweils halbkreisförmige Querschnitte. Es versteht sich, daß auch andere Querschnittsformen möglich sind. Ferner können die Leitungen 46 und 60 - anders als beim Ausführungsbeispiel - unterschiedlich große Querschnittsflächen aufweisen. Weiter sind Schläuche möglich, bei denen mehr als zwei Leitungen vorgesehen sind.

Fig. 3 zeigt einen Querschnitt der Insufflationskanüle 50, bei der es sich beispielsweise um einen Trokar handelt. Auch in der Insufflationskanüle 50 sind zwei Leitungen ausgebildet, nämlich die Zuführ-Leitung 52 sowie die Abführ-Leitung 58. Die Insufflationskanüle 50 besteht beispielsweise aus Metall. Sie hat beim Ausführungsbeispiel eine ähnliche Querschnittsgestalt wie der Schlauch 48, d.h. die beiden Leitungen 52, 58 sind innerhalb eines langgestreckten, im wesentlichen kreiszylindrischen Gehäuses 122 ausgebildet und durch eine Trennwand 124 voneinander getrennt.

Die beiden Leitungen 52, 58 münden vorliegend unmittelbar nebeneinander am vom Schlauch 48 abgewandten Ende der Insufflationskanüle 50. Prinzipiell wäre es aber auch möglich, eine Insufflationskanüle vorzusehen, bei der wie beim Ausführungsbeispiel zwar mehrere Leitungen vorgesehen sind, bei der aber diese Leitungen an relativ weit voneinander entfernten, jedenfalls nicht unmittelbar benachbarten Stellen aus der Insufflationskanüle ausmünden. Beispielsweise könnte wie beim Ausführungsbeispiel der Auslass einer Insufflationsleitung am vorderen Ende einer Insufflationskanüle angeordnet sein, wohingegen ein Einlass oder mehrere Einlässe für eine Meßgasleitung seitlich an der Insufflationskanüle angeordnet sein könnten.

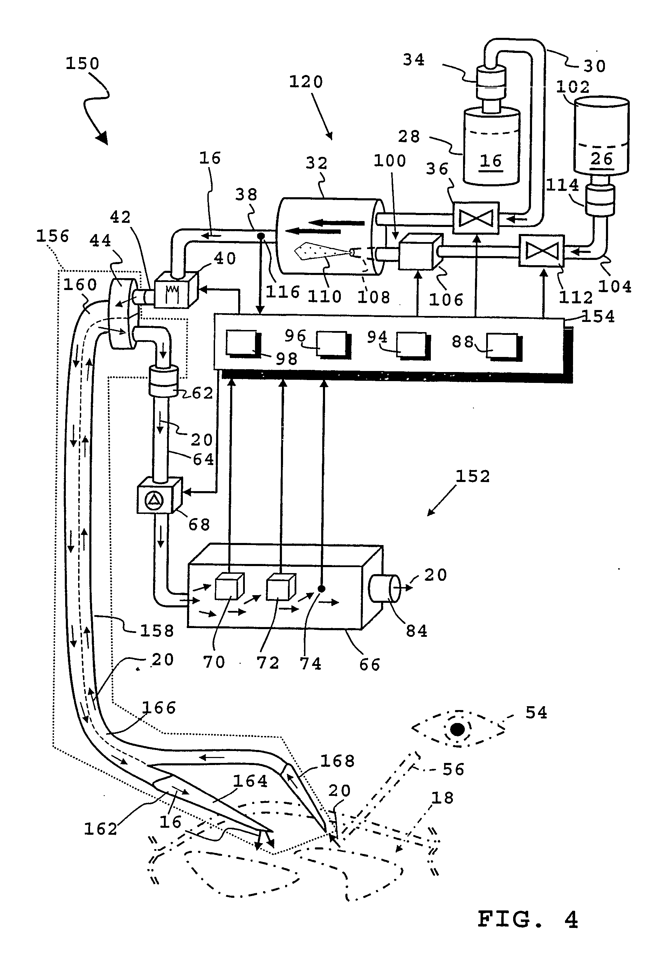

In Fig. 4 ist ein zweites Ausführungsbeispiel einer erfindungsgemäßen Vorrichtung zur Insufflation einer Körperhöhle mit einem Insufflationsgas insgesamt mit der Bezugsziffer 150 versehen.

Die Vorrichtung 150 weist im wesentlichen ähnliche Komponenten auf wie die Vorrichtung 10 gemäß Figuren 1 bis 3. Diejenigen Komponenten, die eine ähnliche Funktion aufweisen, sind in der Fig. 4 mit denselben Bezugsziffern versehen wie in den Figuren 1 bis 3 und werden im folgenden aus Gründen der Vereinfachung nicht näher beschrieben.

Im Gegensatz zur Vorrichtung 10 folgt die Vorrichtung 150 nicht einem modularen Ansatz, sondern bildet eine integrierte Lösung, bei der eine Meßvorrichtung 152 in die Vorrichtung 150 fest integriert ist. Die Vorrichtung 150 weist z.B. ein in der Figur nicht dargestelltes Gehäuse auf, in dem die Meßvorrichtung 152 enthalten ist. Die Meßvorrichtung 152 gleicht ansonsten im wesentlichen der Meßvorrichtung 14, wobei sie im Unterschied zu dieser keine eigene mit der Auswertevorrichtung 76 vergleichbare Auswertevorrichtung aufweist.

Die Funktionen einer Auswertevorrichtung werden bei der Vorrichtung 150 durch eine Steuervorrichtung 154 geleistet, die ansonsten im wesentlichen die Funktionen der Steuervorrichtung 90 erbringt.

Anstelle der Zuführvorrichtung 22 weist die Vorrichtung 150 eine Zu- bzw. Abführvorrichtung 156 auf, die im folgenden vereinfachend "Zuführvorrichtung 156" genannt wird.

Die Zuführvorrichtung 156 enthält eine Schlauch 158, in dem eine Zuführ-Leitung 160 zum Zuführen des Insufflationsgases 16 zu einer Insufflationskanüle 162 vorgesehen ist. In der Insufflationskanüle 162 ist im Unterschied zu der Insufflationskanüle 50 lediglich eine Leitung vorhanden, nämlich eine mit der Zuführ-Leitung 160 verbundene Zuführ-Leitung 164, die am dem Schlauch 158 entgegengesetzten Ende der Insufflationskanüle 162 in die Körperhöhle 18 ausmündet.

Im Schlauch 158 ist neben der Zufuhr-Leitung 160 eine Abführ-Leitung 166 vorhanden, die allerdings nicht mit der Insufflationskanüle 162, sondern mit einer Abführkanüle 168 verbunden ist. Über die Abführkanüle 168, die wie die Insufflationskanüle 162 gemäß der in Figur 4 gezeigten Gebrauchsstellung in der Körperhöhle 18 steckt, kann aus der Körperhöhle 18 ein Gas entnommen werden, das vorliegend das Meßgas 20 bildet. Die Abführkanüle 168 bildet eine Abführvorrichtung. Das Meßgas 20 strömt von der Abführkanüle 168 über die Abführ-Leitung 166 zur Messvorrichtung 152, die das Meßgas 20 in der bereits erläuterten Weise analysiert bzw. für eine kontrollierte Entlüftung der Körperhöhle 18 sorgt.

In diesem Zusammenhang soll erwähnt werden, daß die Meßvorrichtungen 14 und 154 in unterschiedlichen Betriebsmodi betrieben werden können. Sie können z.B. in einem reinen Analysemodus betrieben werden, bei dem verhältnismäßig geringe Mengen Meßgas 20 für eine Analyse genügen und entsprechend geringe Mengen Meßgas 20 aus der Köperhöhle 18 entnommen werden müssen. Möglich ist aber auch ein reiner Entlüftungsmodus, bei dem das Meßgas 20 im Wesentlichen zur Entlüftung der Köperhöhle 19 dient. Es wird verhältnismäßig viel Meßgas 20 aus der Köperhöhle 18 gefördert. Das Meßgas 20 wird dabei nicht oder allenfalls stichprobenhaft analysiert. Besonders bevorzugt ist jedoch ein kombinierter Analyse- und Entlüftungsmodus, bei dem das Meßgas 20 zum einen zur Entlüftung der Köperhöhle 19 dient und zum andern kontiniuierlich oder in kurzen Intervallen analysiert wird.

## Patentansprüche

1. Vorrichtung zur Insufflation einer Körperhöhle (18) mit einem Insufflationsgas (16),
mit einer Insufflationsvorrichtung (12) mit einer Zuführ-Leitung (46; 160) zur Zufuhr eines Insufflationsgases (16) zu der Körperhöhle (18),
**dadurch gekennzeichnet,**
**daß** die Insufflationsvorrichtung (12) eine Abführ-Leitung (60; 166) zum Abführen eines Meßgases (20) aus der Körperhöhle (18) aufweist, und
**daß** sie eine Meßvorrichtung (14; 152) zur Messung eines zusätzlich zu dem Insufflationsgas (16) in dem Meßgas (20) enthaltenen Zusatzstoffes und zur Ausgabe eines Meßsignals (92) in Abhängigkeit von dem Zusatzstoff aufweist, wobei die Meßvorrichtung als Gassensor ausgebildet ist.

2. Vorrichtung nach Anspruch 1, **dadurch gekennzeichnet, daß** die Insufflationsvorrichtung (12) eine Insufflationskanüle (50) aufweist, in der eine Zuführ-Leitung (52) zum Zuführen des Insufflationsgases (16) zu der Körperhöhle (18) und eine Abführ-Leitung (58) zum Abführen des Meßgases (20) aus der Körperhöhle (18) vorhanden ist.

3. Vorrichtung nach Anspruch 1 oder 2, **dadurch gekennzeichnet, daß** die Insufflationsvorrichtung (12) einen Schlauch (48; 158) aufweist, in dem die Zuführ-Leitung (46; 160) zum Zuführen des Insufflationsgases (16) zu der Körperhöhle (18) und die Abführ-Leitung (60; 166) zum Abführen des Meßgases (20) aus der Körperhöhle (18) vorhanden sind.

4. Vorrichtung nach einem der Ansprüche 1 bis 3, **dadurch gekennzeichnet, daß** sie eine Warnvorrichtung (80) zur Ausgabe einer Warnmeldung beim Überschreiten eines vorbestimmten Grenzwertes des von der Meßvorrichtung (14; 152) erfaßten Zusatzstoffes aufweist.

5. Vorrichtung nach einem der vorhergehenden Ansprüche, **dadurch gekennzeichnet, daß** der Gassensor als Gaschromatograph ausgebildet ist.

6. Vorrichtung nach einem der Ansprüche 1 bis 4, **dadurch gekennzeichnet, daß** der Gassensor als Massenspektrometer ausgebildet ist.

7. Vorrichtung nach einem der vorhergehenden Ansprüche, **dadurch gekennzeichnet, daß** der Gassensor zu einer Erfassung, ob das Meßgas (20) brennbar ist, ausgestaltet ist.

8. Vorrichtung nach einem der vorhergehenden Ansprüche, **dadurch gekennzeichnet, daß** der Gassensor zur Erfassung von Methan (CH₄) und/oder Lachgas (N₂O) ausgestaltet ist.

9. Vorrichtung nach einem der vorhergehenden Ansprüche, **dadurch gekennzeichnet, daß** sie eine Druck-Meßvorrichtung (74) zur Messung des Druckes des der Körperhöhle (18) zuzuführenden Insufflationsgases (16) und/oder des Meßgases (20) aufweist.

10. Vorrichtung nach einem der vorhergehenden Ansprüche, **dadurch gekennzeichnet, daß** sie eine Mengen-Meßvorrichtung (72) zur Messung der Menge des Meßgases (20) aufweist.

11. Vorrichtung nach einem der vorhergehenden Ansprüche, **dadurch gekennzeichnet, daß** die Insufflationsvorrichtung (12) zur Steuerung der Menge und/oder des Drucks des der Körperhöhle (18) zuzuführenden Insufflationsgases (16) ausgestaltet ist.

12. Vorrichtung nach einem der vorhergehenden Ansprüche, **dadurch gekennzeichnet, daß** die Insufflationsvorrichtung (12) zur Steuerung der Zufuhr des Insufflationsgases (16) zu der Körperhöhle (18) in Abhängigkeit von dem durch die Meßvorrichtung (14; 152) erzeugten, den Zusatzstoff betreffenden Meßsignal (92) ausgestaltet ist.

13. Vorrichtung nach einem der vorhergehenden Ansprüche, **dadurch gekennzeichnet, daß** die Insufflationsvorrichtung (12) zum Zuführen eines im wesentlichen kontinuierlichen Insufflationsgasstromes zu der Körperhöhle (18) ausgestaltet ist.

14. Vorrichtung nach einem der vorhergehenden Ansprüche, **dadurch gekennzeichnet, daß** die Insufflationsvorrichtung (12) zum Abführen eines im wesentlichen kontinuierlichen Meßgasstromes ausgestaltet ist.

15. Vorrichtung nach einem der vorhergehenden Ansprüche, **dadurch gekennzeichnet, daß** der Meßgasstrom (20) zur Entlüftung der Körperhöhle (18) geeignet ist.

16. Vorrichtung nach einem der vorhergehenden Ansprüche, **dadurch gekennzeichnet, daß** sie eine Abführpumpe (68) zum Abführen des Meßgases (20) aufweist.

## Claims

1. Device for the insufflation of a body cavity (18) with an insufflation gas (16), with an insufflation device (12) with a delivery line (46; 160) for the delivery of an insufflation gas (16) to the body cavity (18),
**characterized in that**
the insufflation device (12) comprises a withdrawl line (60; 166) for the withdrawal of a measurement gas (20) from the body cavity (18), and
that it comprises a measuring device (14; 152) for measuring an additional substance contained in the measurement gas (20) in addition to the insufflation gas (16) and for outputting a measurement signal (92) as a function of the additional substance, whereby the measuring device is designed as a gas sensor.

2. Device according to claim 1, **characterized in that** the insufflation device (12) comprises an insufflation cannula (50) in which there is a delivery line (52) for delivering the insufflation gas (16) to the body cavity (18) and a withdrawal line (58) for withdrawing the measurement gas (20) from the body cavity (18).

3. Device according to claim 1 or 2, **characterized in that** the insufflation device (12) comprises a hose (48; 158) in which there is the delivery line (46; 160) for delivering the insufflation gas (16) to the body cavity (18) and the withdrawal line (60; 166) for withdrawing the measurement gas (20) from the body cavity (18).

4. Device according to any one of claims 1 to 3, **characterized in that** it comprises an alarm device (80) for outputting an alarm message when a predetermined limit value of the additional substance detected by the measuring device (14; 152) is exceeded.

5. Device according to any one of the preceding claims, **characterized in that** the gas sensor is designed as a gas chromatograph.

6. Device according to any one of claims 1 to 4, **characterized in that** the gas sensor is designed as a mass spectrometer.

7. Device according to any one of the preceding claims, **characterized in that** the gas sensor is designed to detect whether the measurement gas (20) is combustible.

8. Device according to any one of the preceding claims, **characterized in that** the gas sensor is designed to detect methane (CH₄) and/or nitrous oxide (N₂O).

9. Device according to any one of the preceding claims, **characterized in that** it comprises a pressure measuring device (74) for measuring the pressure of the insufflation gas (16) to be delivered to the body cavity (18) and/or of the measurement gas (20).

10. Device according to any one of the preceding claims, **characterized in that** it comprises a quantity measuring device (72) for measuring the quantity of the measurement gas (20).

11. Device according to any one of the preceding claims, **characterized in that** the insufflation device (12) is designed to control the quantity and/or the pressure of the insufflation gas (16) to be delivered to the body cavity (18).

12. Device according to any one of the preceding claims, **characterized in that** the insufflation device (12) is designed to control the delivery of the insufflation gas (16) to the body cavity (18) as a function of the measurement signal (92) generated by the measuring device (14; 152) and relating to the additional substance.

13. Device according to any one of the preceding claims, **characterized in that** the insufflation device (12) is designed to deliver a substantially continuous stream of insufflation gas to the body cavity (18).

14. Device according to any one of the preceding claims, **characterized in that** the insufflation device (12) is designed to withdraw a substantially continuous stream of measurement gas.

15. Device according to any one of the preceding claims, **characterized in that** the measurement gas stream (20) is suitable for venting the body cavity (18).

16. Device according to any one of the preceding claims, **characterized in that** it comprises a withdrawal pump (68) for withdrawing the measurement gas (20).

## Revendications

1. Dispositif pour l'insufflation d'un gaz d'insufflation (16) dans une cavité corporelle (18),
avec un dispositif d'insufflation (12) comportant une conduite d'alimentation (46 ; 160) pour acheminer un gaz d'insufflation (16) dans la cavité corporelle (18),
**caractérisé en ce que**,
le dispositif d'insufflation (12) présente une conduite d'évacuation (60 ; 166) pour évacuer un gaz mesuré (20) de la cavité corporelle (18), et
il présente un dispositif de mesure (14; 152) pour mesurer un additif contenu dans le gaz mesuré (20) en plus du gaz d'insufflation (16) et pour émettre un signal de mesure (92) en fonction de l'additif, le dispositif de mesure étant réalisé sous la forme d'un capteur de gaz.

2. Dispositif selon la revendication 1, **caractérisé en ce que** le dispositif d'insufflation (12) présente une canule d'insufflation (50), dans laquelle une conduite d'alimentation (52) est présente pour acheminer le gaz d'insufflation (16) dans la cavité corporelle (18) et une conduite d'évacuation (58) est présente pour évacuer le gaz mesuré (20) de la cavité corporelle (18).

3. Dispositif selon la revendication 1 ou 2, **caractérisé en ce que** le dispositif d'insufflation (12) présente un tube ou tuyau (48 ; 158), dans lequel la conduite d'alimentation (46 ; 160) est présente pour acheminer le gaz d'insufflation (16) dans la cavité corporelle (18) et la conduite d'évacuation (60 ; 166) est présente pour évacuer le gaz mesuré (20) de la cavité corporelle (18).

4. Dispositif selon l'une des revendications 1 à 3, **caractérisé en ce qu'**il présente un dispositif d'alarme ou d'alerte (80) pour émettre un signal d'alarme en cas de dépassement d'une valeur seuil prédéterminée pour la additif détectée par le dispositif de mesure (14 ; 152).

5. Dispositif selon l'une quelconque des revendications précédentes, **caractérisé en ce que** le capteur de gaz est formé par un chromatographe en phase gazeuse.

6. Dispositif selon l'une des revendications 1 à 4, **caractérisé en ce que** le capteur de gaz est formé par un spectromètre de masse.

7. Dispositif selon l'une quelconque des revendications précédentes, **caractérisé en ce que** le capteur de gaz est configuré de façon à détecter si le gaz mesuré (20) est inflammable.

8. Dispositif selon l'une quelconque des revendications précédentes, **caractérisé en ce que** le capteur de gaz est configuré pour détecter du méthane (CH₄) et/ou du gaz hilarant (N₂O).

9. Dispositif selon l'une quelconque des revendications précédentes, **caractérisé en ce qu'**il présente un dispositif de mesure de la pression (74) pour mesurer la pression du gaz d'insufflation (16) à acheminer dans la cavité corporelle (18) et/ou du gaz mesuré (20).

10. Dispositif selon l'une quelconque des revendications précédentes, **caractérisé en ce qu'**il présente un dispositif de mesure de la quantité (72) pour mesurer la quantité du gaz mesuré (20).

11. Dispositif selon l'une quelconque des revendications précédentes, **caractérisé en ce que** le dispositif d'insufflation (12) est configuré pour contrôler la quantité et/ou la pression du gaz d'insufflation (16) à acheminer dans la cavité corporelle (18).

12. Dispositif selon l'une quelconque des revendications précédentes, **caractérisé en ce que** le dispositif d'insufflation (12) est configuré pour contrôler l'alimentation en gaz d'insufflation (16) dans la cavité corporelle (18) en fonction du signal de mesure (92) concernant l'additif émis par le dispositif de mesure (14 ; 152).

13. Dispositif selon l'une quelconque des revendications précédentes, **caractérisé en ce que** le dispositif d'insufflation (12) est configuré pour acheminer un flux de gaz d'insufflation essentiellement continu dans la cavité corporelle (18).

14. Dispositif selon l'une quelconque des revendications précédentes, **caractérisé en ce que** le dispositif d'insufflation (12) est configuré pour l'évacuation d'un flux essentiellement continu de gaz mesuré.

15. Dispositif selon l'une quelconque des revendications précédentes, **caractérisé en ce que** le flux de gaz mesuré (20) est approprié pour la ventilation de la cavité corporelle (18).

16. Dispositif selon l'une quelconque des revendications précédentes, **caractérisé en ce qu'**il présente une pompe d'évacuation (68) pour évacuer le gaz mesuré (20).
